(19) Europäisches Patentamt
European Patent Office
Office européen des brevets

(11) Publication number: 0 179 447 B1

(12) **EUROPEAN PATENT SPECIFICATION**

(45) Date of publication of patent specification: 19.07.89

(51) Int. Cl.[4]: C 07 C 43/225, C 07 C 41/18

(21) Application number: 85113402.3

(22) Date of filing: 22.10.85

(54) **Process for the synthesis of 2-methoxy-6-bromo-naphthalene.**

(30) Priority: 23.10.84 IT 2328084

(43) Date of publication of application: 30.04.86 Bulletin 86/18

(45) Publication of the grant of the patent: 19.07.89 Bulletin 89/29

(84) Designated Contracting States: BE DE FR GB

(56) References cited: DE-A-2 619 641

(73) Proprietor: Montedison S.p.A.
31, Foro Buonaparte
I-20121 Milan (IT)

(72) Inventor: Borsotti, Giampiero
14, via Pastore
Novara (IT)

(74) Representative: Zumstein, Fritz jun., Dr. et al
Dr. F. Zumstein sen. Dr. E. Assmann Dr. R. Koenigsberger Dipl.-Ing. F. Klingseisen Dr. F. Zumstein jun. Bräuhausstrasse 4
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 179 447 B1

## Description

The invention relates to a process for the synthesis of 2-methoxy-6-bromo-naphthalene having the following formula:

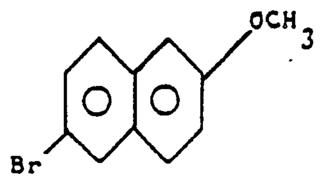

(I)

which is an intermediate in the preparation of an anti-inflammatory product known by the trademark Naproxen® or Naproxyn®. The compound of formula (I) can be obtained by bromination of β-naphthol to 1,6-dibromo-β-naphthol, by dehalogenation of the latter to 6-bromo-β-naphthol by means of metallic tin and, lastly, by alkylation with dimethylsulphate or methanol, in the presence of acid catalysts. Tin, however, is a metal that is not easily available and, therefore, this method is of little interest from an industrial point of view. A second, even more complicated, method is described in German patent 2619641. The process is based on dehalogenation by means of Sn and, if it is applied to 1,6-dibromo-2-methoxy-naphthalene, does not give a high yield because, under the reaction conditions, the methoxy group is easily hydrolyzed and remarkable amounts of the undesired product, 2-hydroxy-6-bromo-naphthalene, are formed.

Therefore, an object of the invention is to provide a simpler process by which one can directly obtain a high yield of 2-methoxy-6-bromo-naphthalene under very mild conditions and within a short time.

It has been found that the above-mentioned objects can easily be achieved by bromination of 2-methoxy-naphthalene in a solution of acetic or propionic acid to 1,6-dibromo-2-methoxy-naphthalene and subsequent debromination of the latter, without any separation from the liquid reaction medium, by adding metallic iron (for instance in the form of powder or chips).

Accordingly the invention is directed to a process for the manufacture of 2-methoxy-6-bromo-naphthalene, wherein 2-methyl-naphthalene is brominated by means of a solution of bromine in acetic or propionic acid and wherein the thus obtained 1,6-dibromo-2-methoxy-naphthalene is debrominated, without any separation from the liquid reaction medium, by addition of at least one mole of iron per mole of initial 2-methoxy-naphthalene, the amount of HBr in the liquid reaction medium being equal to or higher than 2 moles per mole of dibromo derivative.

This process can be schematically represented by the following equations:

1) 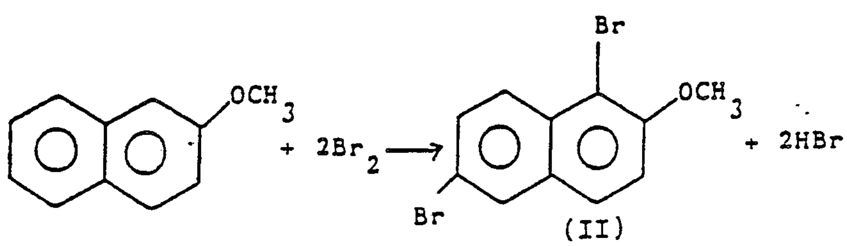


(II)

2) 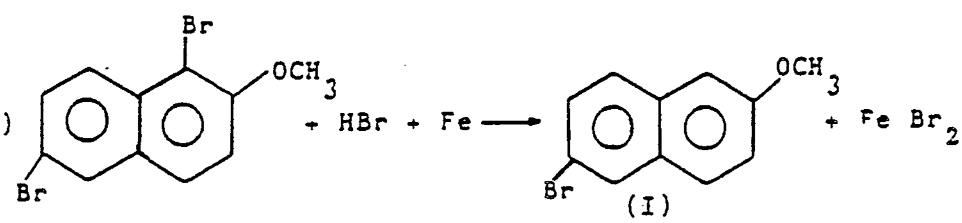


(I)

The invention can be carried out in different ways. According to a preferred way, the process comprises the following steps, all of them to be carried out with one apparatus only and in one medium only:

A) Bromine, as such or diluted with $CH_3COOH$ or propionic acid, is added under stirring, at 30—50°C, to a solution of 2-methoxy-naphthalene in acetic or propionic acid, trying carefully to keep the formed hydrobromic acid in the liquid phase (during addition and during the subsequent operations), and adjusting, if necessary, the temperature by heat exchange (indirectly by means of a refrigerant fluid, for instance water);

B) The stirring is continued at temperatures ranging between 30 and 50°C until the 2-methoxy-naphthalene disappears;

C) Iron is added in the form of powder or chips, the temperature, under stirring, being kept at between 30 and 60°C, provided the amount of hydrobromic acid in the liquid medium of reaction is at least equal to 2 moles per mole of dibromo derivative; the temperature can be higher than 60°C as well, provided the liquid medium of reaction is always saturated with HBr;

D) After the reaction mixture has been diluted, the 2-methoxy-6-bromo-naphthalene is separated by filtration, decantation, centrifugation or extraction;

E) The 2-methoxy-6-bromo-naphthalene is purified by crystallization from suitable solvents such as, for instance, aliphatic alcohols having from 1 to 4 C atoms or aliphatic hydrocarbons such as cyclohexane, heptane and isooctane.

If iron is added in the form of powder, the amount of iron substantially has to be equal to at least 1 mole per mole of 2-methoxy-naphthalene added at the start. In the case of chips, however, it is better to increase the lowest level to at least 3 moles/mole. Surprisingly, only iron among the most commonly available metals proves to be effective for the above-mentioned debromination. The use of similar, conventional metals such as Al, Mg and Zn, is ineffective and only leads to the formation of hydrogen. When using iron, however, no development of hydrogen, the danger and undesirability of which is well known, was found.

The following examples illustrate the invention without, however, limiting its scope.

Example 1

Over a period of 35 minutes a solution of 81 g bromine in 25 $cm^3$ $CH_3COOH$ was added to a well-stirred suspension of 39.25 g of 2-methoxy-naphthalene in 125 $cm^3$ of glacial $CH_3COOH$ heated to 30°C, and the temperature kept at 40 to 45°C, avoiding that the formed hydrobromic acid leaves the reaction medium; after the bromine had been added, the mixture was stirred for 1.5 h at 45°C in order to complete the reaction. 14 g of iron in the form of powder were then added in small doses over a period of 1.5 hours and, at intervals, the exothermal reaction was slowed down by means of a cooling bath. Stirring was continued until 1,6-dibromo-2-methoxy-naphthalene disappeared (on thin layer chromatography) and then the mixture was diluted with 0.5 liters of $H_2O$. The compound was then filtered, washed with $H_2O$, and dissolved in $CH_2Cl_2$. Finally, the chloromethylenic solution was washed with 5% NaOH, dehydrated and evaporated. 55.2 g of raw 2-methoxy-6-bromo-naphthalene were obtained. After a crystallization from 250 $cm^3$ of isobutanol 45 g of pure product were obtained (gas-chromatographic analysis) having a melting point of between 105 and 106°C.

Example 2

Example 1 was repeated but the step of dissolving the compound in methylene chloride again was left out. Essentially the same results (after crystallization from isobutanol) were obtained.

Example 3

Example 2 was repeated, but the iron powder replaced by iron chips. Substantially the same results were obtained.

Example 4

Example 2 was repeated, but the acetic acid replaced by an equivalent amount of propionic acid. Substantially the same results were obtained.

Example 5

Example 2 was repeated, but the isobutanol replaced by n-heptane. Excellent results were obtained.

**Claims**

1. A process for the manufacture of 2-methoxy-6-bromo-naphthalene, wherein 2-methoxy-naphthalene is brominated by means of a solution of bromine in acetic or propionic acid and wherein the thus obtained 1,6-dibromo-2-methoxy-naphthalene is debrominated, without any separation from the liquid reaction medium, by addition of at least one mole of iron per mole of initial 2-methoxy-naphthalene, the amount of HBr in the liquid reaction medium being equal to or higher than 2 moles per mole of dibromo derivative.

2. A process according to Claim 1, wherein iron is added in the form of powder.

3. A process according to Claim 1, wherein iron is added in the form of chips, the amount of iron being at least 3 moles per mole of initial 2-methoxy-naphthalene.

4. A process according to any one of Claims 1 to 3, wherein the synthesis is carried out in acetic acid or propionic acid.

5. A process according to any one of Claims 1 to 4, wherein the temperature is ranging from 30 to 60°C.

6. A process according to any one of Claims 1 to 5, wherein a pure product is obtained by distillation or crystallization from suitable solvents, in particular from aliphatic alcohols and hydrocarbons, preferably isobutanol or n-heptane.

**Patentansprüche**

1. Verfahren zur Herstellung von 2-Methoxy-6-bromnaphthalin, bei dem 2-Methoxynaphthalin mit einer Lösung von Brom in Essigsäure oder Propionsäure bromiert, und das so erhaltene 1,6-Dibrom-2-methoxynaphthalin ohne Abtrennung aus dem flüssigen Reaktionsmedium durch Zugabe von zumindest einem Mol Eisen je Mol anfängliches 2-Methoxynaphthalin debromiert wird, wobei die Menge an HBr in dem flüssigen Reaktionsmedium gleich oder höher als 2 Mol je Mol Dibromderivat ist.

2. Verfahren gemäß Anspruch 1, bei dem Eisen in Form von Pulver zugegeben wird.

3. Verfahren gemäß Anspruch 1, bei dem Eisen in Form von Chips zugegeben wird, wobei die Menge des Eisens zumindest 3 Mol je Mol anfängliches 2-Methoxynaphthalin beträgt.

4. Verfahren gemäß einem der Ansprüche 1 bis 3, bei dem die Synthese in Essigsäure oder Propionsäure durchgeführt wird.

5. Verfahren gemäß einem der Ansprüche 1 bis 4, bei dem die Temperatur im Bereich von 30 bis 60°C liegt.

6. Verfahren gemäß einem der Ansprüche 1 bis 5, bei dem ein reines Produkt durch Destillation oder Kristallisation aus geeigneten Lösungsmitteln, insbesondere aus aliphatischen Alkoholen und Kohlenwasserstoffen, vorzugsweise Isobutanol oder n-Heptan, erhalten wird.

**Revendications**

1. Un procédé pour la fabrication de 2-méthoxy-6-bromo-naphtalène, dans lequel le 2-méthoxy-naphtalène est bromé à l'aide d'une solution de brome dans l'acide acétique ou propionique et, dans laquelle le 1,6-dibromo-2-méthoxy-naphtalène ainsi obtenu est débromé, sans qu'il ne soit nécessaire de le séparer du milieu réactionnel liquide, par addition d'au moins 1 mole de fer par mole de 2-méthoxy-naphtalène initialement employé, la quantité de HBr dans le milieu réactionnel liquide étant au moins égale à 2 moles par mole de dérivé dibromo.

2. Un procédé selon la revendication 1, dans lequel le fer est ajouté sous forme pulvérulante.

3. Un procédé selon la revendication 1, dans lequel le fer est ajouté sous forme de copeaux, la quantité de fer étant d'au moins 3 moles par mole de 2-méthoxy-naphtalène initial.

4. Un procédé selon l'une quelconque des revendications 1 à 3, dans lequel la synthèse est mise en oeuvre dans l'acide acétique ou l'acide propionique.

5. Un procédé selon l'une quelconque des revendications 1 à 4, dans lequel la température est comprise entre 30 et 60°C.

6. Un procédé selon l'une quelconque des revendications 1 à 5, dans lequel on obtient un produit pur par distillation ou cristallisation dans des solvants convenables notamment des alcools ou des hydrocarbures aliphatiques, de préférence, l'isobutanol ou du n-heptane.